# FASCICULE DE BREVET EUROPEEN

(11) **EP 2 550 094 B1**
(45) Date de publication et mention de la délivrance du brevet: **23.04.2014**
(21) Numéro de dépôt: 11715720.6
(22) Date de dépôt: 23.03.2011
(51) Int. Cl.: B01J 19/24, C07C 227/08, C07C 231/12

(54) **PROCEDE CONTINU DE FABRICATION DE SOLUTIONS AQUEUSES DE BETAÏNE**
KONTINUIERLICHES VERFAHREN ZUR HERSTELLUNG VON WÄSSRIGEN BETAINLÖSUNGEN
CONTINUOUS METHOD FOR MANUFACTURING BETAINE AQUEOUS SOLUTION

(30) Priorité: 23.03.2010 FR 1052079
(43) Date de publication de la demande: 30.01.2013
(73) Titulaire: Rhodia Operations, 93306 Aubervilliers (FR)
(72) Inventeur: LOMEL, Sébastien, F-38540 Saint Just Chaleyssin (FR); PITIOT, Pascal, F-69008 Lyon (FR); GISBERT, Thierry, F-01700 Miribel (FR)
(74) Mandataire: Domenego, Bertrand
(86) Numéro de dépôt international: PCT/FR2011/050611
(87) Numéro de publication internationale: WO 2011/117535

(56) Documents cités:
- US-A- 4 497 825
- US-A- 5 292 942

## Description

La présente invention a pour objet un procédé en continu de fabrication de solutions aqueuses de bétaïne.

Le glyphosate (N-(phosphonométhyl)glycine, C₃H₈NO₅P) est un herbicide bien connu de l'homme du métier. Il existe différentes formulations comprenant cet herbicide. Il est notamment particulièrement intéressant de disposer pour l'utilisateur final (notamment l'agriculteur) de compositions prêtes à l'emploi.

Cet herbicide est notamment présenté sous la forme de compositions comprenant du glyphosate, de l'eau et au moins un agent tensioactif.

Actuellement, on connaît essentiellement des compositions comprenant du glyphosate et des tensioactifs de la famille des amines grasses éthoxylées. Toutefois, ces tensioactifs sont connus comme étant écotoxiques, irritants et faiblement biodégradables.

Ainsi, ces tensioactifs sont maintenant remplacés par d'autres tensioactifs, notamment par des composés d'ammonium quaternaire de la famille des bétaïnes.

La préparation de solutions aqueuses de bétaïnes par réaction d'amines tertiaires avec un acide ohalocarboxylique et une base en phase aqueuse est bien connue de l'homme du métier, et notamment des brevets US 3 819 539 et US 4 497 825.

Les solutions aqueuses résultantes comprennent essentiellement la bétaïne, le sel d'halogène de métal alcalin formé et de l'eau.

Ces solutions sont utilisables en l'état. Toutefois, si elles contiennent une quantité résiduelle trop importante d'amine, les propriétés de ces solutions sont amoindries.

Le brevet US 5 292 942 décrit un procédé de préparation de solutions aqueuses de bétaine selon un procédé continu dans au moins deux réacteurs agités. Toutefois, ce procédé est un procédé long avec des temps de séjour des réactifs trop importants, supérieurs à 10 heures, ce qui ne permet pas de récupérer rapidement le produit final. Les réacteurs agités adoptés dans ce brevet se caractérisent par une distribution du temps de séjour étalée qui nécessite lors d'un changement de production, en termes de débits, de nature de bétaïne produite ou autres, d'attendre au moins trois fois le temps de séjour de chaque réacteur pour retrouver une qualité admissible du produit. Aussi, dans le brevet cité, il est nécessaire d'attendre au moins 50 heures pour produire un produit de premier choix, la production durant l'intercampagne étant déclassée et détruite. L'adoption de long temps de séjour conduit, pour un débit donné, à dimensionner des unités qui sont importantes en volume, que l'on ne peut envisager de transporter, et qui sont coûteuses d'un point de vue investissement.

La présente invention a pour but de fournir un procédé de préparation de solutions aqueuses de bétaïnes présentant des temps de séjour réduits.

La présente invention a également pour but de fournir un procédé de préparation flexible et compact, permettant d'obtenir des solutions aqueuses de bétaïnes de qualité constante sans étape de correction de la qualité.

La présente invention a également pour but de fournir un procédé de préparation de solutions aqueuses de bétaïnes se caractérisant par la possibilité de réaliser les étapes de formulation de la bétaïne en continu et ainsi de s'affranchir de toute étape discontinue de mélange ultérieure et également de stockage.

La présente invention a également pour but de fournir un procédé rapide et simple pour préparer des solutions aqueuses de bétaïnes avec une concentration élevée en bétaïne.

La présente invention concerne un procédé de préparation en continu d'une solution aqueuse de bétaïne de formule (I) suivante : 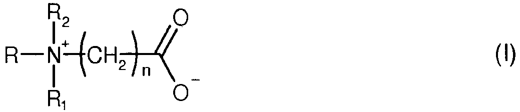 dans laquelle :
- n est égal à 1, 2 ou 3 ;
- R₁ représente un groupe alkyle, linéaire ou ramifié, comprenant de 1 à 3 atomes de carbone, de préférence un groupe méthyle ;
- R₂ représente un groupe alkyle, linéaire ou ramifié, comprenant de 1 à 3 atomes de carbone, de préférence un groupe méthyle ;
- R représente :
   - soit une chaîne hydrocarbonée, linéaire ou ramifiée, de préférence un groupe alkyle, comprenant de 3 à 30 atomes de carbone, et notamment de 3 à 20 atomes de carbone ;
   - soit un groupe -A-NH-CO-R₃,
      R₃ représentant une chaîne hydrocarbonée, linéaire ou ramifiée, de préférence un groupe alkyle, comprenant de 3 à 30 atomes de carbone, et notamment de 3 à 20 atomes de carbone ; et
      A représentant un groupe hydrocarboné divalent, linéaire ou ramifié, comprenant de 1 à 6 atomes de carbone, éventuellement substitué par un groupe hydroxyle, et de préférence choisi parmi les groupes : -CH₂-CH₂-CH₂- et -CH₂-CHOH-CH₂- ;
comprenant la réaction d'une amine de formule NRR¹R², R, R¹ et R² étant tels que définis ci-dessus, avec un acide ω-halocarboxylique de formule X-(CH₂)ₙ-COOH, X représentant un atome d'halogène et n étant tel que défini ci-dessus, en présence d'eau et d'une base, notamment d'un hydroxyde de métal alcalin, et plus particulièrement KOH ou NaOH,
caractérisé en ce que ledit procédé est effectué dans un dispositif constitué d'au moins deux réacteurs successifs (R1) et (R2), le réacteur (R2) étant un réacteur tubulaire.

Selon la présente invention, le terme "chaîne hydrocarbonée" désigne un groupe comprenant des atomes de carbone et des atomes d'hydrogène, et désigne plus particulièrement les groupes alkyles.

Selon la présente invention, les radicaux "alkyle" représentent des radicaux hydrocarbonés saturés, en chaîne droite ou ramifiée, comprenant par exemple de 1 à 3 atomes de carbone, ou encore de 3 à 30, de préférence de 3 à 20, atomes de carbone (ils peuvent typiquement être représentés par la formule CₙH₂ₙ₊₁, n représentant le nombre d'atomes de carbone). On peut notamment citer, lorsqu'ils sont linéaires, les radicaux méthyle, éthyle, propyle, butyle, pentyle, hexyle, octyle, nonyle et décyle. On peut notamment citer, lorsqu'ils sont ramifiés ou substitués par un ou plusieurs radicaux alkyles, les radicaux isopropyle, tert-butyle, 2-éthylhexyle, 2-méthylbutyle, 2-méthylpentyle, 1-méthylpentyle et 3-méthylheptyle.

Dans la formule (I), les groupes R₁ et R₂ peuvent être identiques ou différents.

De préférence, dans la formule (I), R représente un groupe alkyle comprenant 12 ou 14 atomes de carbone.

Parmi les atomes d'halogène, pour la définition de X, on cite plus particulièrement les atomes de fluor, de chlore, de brome et d'iode. De préférence, X représente un atome de chlore.

Le procédé de la présente invention consiste donc à préparer des solutions aqueuses de bétaïne, c'est-à-dire des solutions comprenant au moins de la bétaïne (répondant à la formule (I) ci-dessus) et de l'eau.

Contrairement aux procédés souvent mis en oeuvre pour la préparation de solutions de bétaïne, qui sont des procédés discontinus (ou procédés batch), le procédé de l'invention est effectué en continu. Ainsi, le procédé de l'invention consiste à effectuer toutes les étapes de façon continue.

Par exemple, les réactifs, à savoir notamment l'amine et l'acide, sont introduits dans le dispositif de façon continue, et non, comme dans le cadre d'un procédé discontinu, en une seule fois.

Le procédé de la présente invention consiste notamment à faire réagir l'amine de formule (I) avec un acide ω-halocarboxylique, cette réaction étant bien connue de l'homme du métier.

Cette réaction est effectuée en présence d'une base qui va permettre de se trouver dans des conditions appropriées de pH et donc l'acide ω-halocarboxylique se trouvera sous sa forme carboxylate.

Parmi les bases utilisées pour la réaction entre l'amine et l'acide, on peut notamment citer KOH ou NaOH, ou encore toute base forte telle que LiOH, CsOH ou Ca(OH)₂. De préférence, la base utilisée est la potasse.

L'amine utilisée dans le cadre du procédé de la présente invention peut être sous la forme d'un mélange de plusieurs amines.

De même, l'acide utilisé dans le cadre du procédé de la présente invention peut être sous la forme d'un mélange de plusieurs acides.

Le dispositif mis en oeuvre dans le cadre de la présente invention comprend donc au moins deux réacteurs, et de préférence deux réacteurs (R1) et (R2).

Ces deux réacteurs successifs sont disposés en cascade, c'est-à-dire l'un à la suite de l'autre. Ainsi, la sortie du réacteur (R1) est reliée à l'entrée du réacteur (R2).

Le réacteur (R1), situé en amont, peut être de nature quelconque, c'est-à-dire un réacteur agité ou un réacteur tubulaire, et le réacteur (R2) est un réacteur tubulaire.

Selon le procédé de la présente invention, l'amine et l'acide ω-halocarboxylique sont introduits de façon continue dans le réacteur (R1) et réagissent ensemble pour former la solution aqueuse de bétaïne récupérée à la sortie du réacteur (R2). De même, le mélange réactionnel (M), récupéré à la sortie du réacteur (R1), est introduit en continu, c'est-à-dire au fur et à mesure de l'avancement de la réaction, à l'entrée du réacteur (R2).

Selon un mode de réalisation préféré, le procédé de la présente invention comprend :
a) une étape d'introduction, à l'entrée du réacteur (R1), de l'eau, de la base, de l'amine et de l'acide ω-halocarboxylique ;
b) une étape de réaction entre l'amine et l'acide ohalocarboxylique dans le réacteur (R1), pour obtenir, à la sortie du réacteur (R1), un mélange réactionnel aqueux (M) comprenant de la bétaïne, de l'amine et de l'acide ω-halocarboxylique ;
c) une étape d'introduction du mélange réactionnel aqueux (M) à l'entrée du réacteur (R2), pour poursuivre la réaction entre l'amine et l'acide ω-halocarboxylique ; et
d) une étape de récupération d'une solution aqueuse de bétaïne (M') à la sortie du réacteur (R2) comprenant de la bétaïne à raison d'au moins 30% en poids, moins de 2,5% en poids, de préférence moins de 1,65% en poids, d'amine, et moins de 1,5% en poids, notamment moins de 1% en poids, et de préférence moins de 0,7% en poids, d'acide.

Le mélange réactionnel (M) comprend majoritairement de la bétaïne, et de préférence à raison d'au plus 30% en poids par rapport au poids total du mélange. Il comprend également de l'amine et de l'acide ω-halocarboxylique résiduels. L'amine est présente dans le mélange (M) à raison de 3% à 6%, et de préférence de 5%, en poids, et l'acide est présent à raison de 2% à 5%, et de préférence de 2%, en poids.

Ce mélange contient également de nombreux produits issus des réactions secondaires, et notamment de l'acide glycolique.

Selon un mode de réalisation particulier, le mélange (M) comprend 5% en poids d'amine, 2% en poids d'acide, 1% en poids d'acide glycolique et 26% en poids de bétaïne.

Le mélange réactionnel (M') comprend de la bétaïne à raison d'au moins 30% en poids, préférentiellement de 30% à 50% en poids. Selon un mode de réalisation particulier, la quantité de bétaïne dans ce mélange (M') est d'environ 34% en poids par rapport au poids total de la composition.

Toutes les étapes du procédé de l'invention sont effectuées en continu.

Lors de l'étape a), l'eau peut également être introduite via une dilution de l'acide. Dans ce cas, cette étape d'introduction se fait par l'intermédiaire de trois flux d'entrée, à savoir une entrée d'amine, d'acide dilué et de base.

Le mélange réactionnel aqueux (M), obtenu à la sortie du réacteur (R1), et introduit dans le réacteur (R2), comprend de l'eau, de l'amine de formule (I), de l'acide ω-halocarboxylique et la bétaïne formée dans le réacteur (R1).

Ce mélange réactionnel n'est pas utilisable en l'état en raison de quantités encore trop importantes d'amine et d'acide résiduels.

Selon l'avancement de la réaction, la concentration en bétaïne augmente au cours du temps dans le réacteur (R2).

Selon un mode de réalisation particulièrement préféré, lors de l'étape a) du procédé de l'invention, on introduit, d'une part, l'amine avec la base, et, d'autre part, l'acide avec l'eau.

De préférence, l'amine et la base sont mélangées et introduites ensemble, par exemple via des mélangeurs ou micromélangeurs.

De préférence, l'acide et l'eau sont mélangés et introduits ensemble, par exemple via des mélangeurs ou micromélangeurs.

Ce mode de réalisation permet d'éviter la réaction exothermique de neutralisation qui a lieu entre l'acide et la base. En effet, cette réaction exothermique peut entraîner des problèmes de cristallisation liée à la faible solubilité du sel en fonction de la température. Ce mode de réalisation permet également d'éviter des quantités importantes de produits secondaires dont les réactions sont activées par l'élévation de température.

Selon un mode de réalisation particulièrement préféré, le procédé de la présente invention peut comprendre une étape supplémentaire b'), consistant à ajouter de la base dans le mélange réactionnel (M) à la sortie du réacteur (R1) et avant l'introduction dudit mélange dans le réacteur (R2). Cette étape est donc effectuée entre l'étape b) et l'étape c). Cet ajout de base peut également être effectué directement dans le réacteur (R2).

Cette base peut être la même que la base utilisée dans le réacteur (R1) mais elle peut aussi être différente.

Cette étape supplémentaire permet ainsi de contrôler le pH au cours de la réaction dans (R1) et (R2) et donc d'augmenter la vitesse de réaction et limiter les réactions secondaires. De préférence, le pH à la sortie du réacteur (R1) est supérieur à 7.

Le procédé de l'invention présente également l'avantage de ne pas nécessiter d'étape supplémentaire d'ajout d'acide chlorhydrique dans la solution (M'), dans la mesure où le pH du milieu à la sortie du réacteur est compris entre 7 et 8.

L'ajout de la quantité de base nécessaire exacte (par ajustement grâce à l'injection secondaire de base entre (R1) et (R2)) évite d'avoir besoin de corriger le pH en sortie de (R2), ce dernier étant compris entre 7 et 8. A titre de comparaison, en réacteur batch, le pH avant addition de HCl est de 10-11 environ.

Un autre mode de réalisation préféré du procédé de l'invention consiste à réinjecter une portion de la solution aqueuse de bétaïne (M') à l'entrée du réacteur (R1), en mélange avec l'amine et la base. Ainsi, on peut de préférence récupérer de la solution aqueuse de bétaïne (M') à la sortie du réacteur (R2) pour la réintroduire à l'entrée du réacteur (R1). On réinjecte de préférence 1% en poids de la solution (M') par rapport au débit total.

Ce mode de réalisation permet de faciliter la solubilisation de l'amine et permet d'éviter la décantation du milieu réactionnel qui est à l'origine diphasique.

Selon un autre mode de réalisation du procédé, la sortie du réacteur (R2) peut être reliée à un ou plusieurs mélangeurs, en série ou en parallèle, permettant l'addition et le mélange en continu d'additifs tels que l'eau, la glycérine, le propylène, le sorbitol, les glycols, tout sucre non réducteur, des agents anti-mousse ou tout autre additif liquide nécessaire à la formulation, ou des mélanges de ceux-ci, afin de préparer des formulations en ligne à base de bétaïne.

Le procédé de l'invention permet d'obtenir des solutions aqueuses de bétaïne utilisables en l'état, c'est-à-dire pouvant être utilisées directement pour la préparation de formulations, notamment pour des formulations de compositions herbicides, et plus particulièrement pour des formulations à base de glyphosate.

Pour ce faire, le dispositif mis en oeuvre dans le cadre de la présente invention peut être relié à des mélangeurs comprenant les composés appropriés pour la préparation des formulations souhaitées. Ainsi, la sortie du réacteur (R2) peut être reliée par exemple à un mélangeur comprenant de l'eau et à un mélangeur comprenant de la glycérine.

Les formulations peuvent alors être directement livrées aux utilisateurs dans des camions. Ceci évite d'utiliser différentes installations. On peut ainsi relier directement le dispositif de l'invention au camion qui sera utilisé pour livrer la formulation à l'utilisateur final.

Les formulations préparées à partir des solutions aqueuses obtenues selon le procédé de l'invention peuvent également comprendre d'autres agents tensioactifs, des agents anti-mousse, des solvants, de préférence des solvants miscibles à l'eau.

De préférence, la température à l'intérieur du réacteur (R1) est comprise de 80 °C à 110 °C, de préférence de 90 °C à 100 °C, et préférentiellement égale à environ 95°C.

Le choix de ces gammes de températures résulte d'un compromis entre promouvoir rapidement la réaction principale tout en essayant de limiter les réactions secondaires dans un premier temps.

De préférence, la température à l'intérieur du réacteur (R2) est comprise de 95°C à 110°C, de préférence de 100°C à 105°C, et préférentiellement égale à environ 105°C.

La température est augmentée dans (R2) de façon cette fois-ci à favoriser la réaction secondaire, tout en accélérant également la réaction principale, le but *in fine* étant d'être dans les spécifications pour l'acide et l'amine résiduels, c'est-à-dire dans les teneurs souhaitées en acide et amine dans la solution aqueuse de bétaïne finale.

De préférence, la pression à l'intérieur du réacteur (R2) est comprise de 1 à 10 bars, de préférence de 1,5 bars à 5 bars, et préférentiellement de 2 à 3 bars.

La pression est choisie de façon à ce que la température d'ébullition du milieu réactionnel soit supérieure à la température de travail, si bien que le milieu reste liquide et homogène. La pression peut également être choisie de façon à ce qu'elle soit supérieure à la pression de vapeur saturante du milieu à la température de travail.

Selon un mode de réalisation particulièrement préféré, le réacteur (R1) est un réacteur tubulaire.

Le procédé de la présente invention est donc de préférence effectué dans un dispositif constitué de deux réacteurs tubulaires (R1) et (R2).

Selon un mode de réalisation préféré du procédé de l'invention, le temps de séjour de l'amine et de l'acide dans le réacteur (R1) est compris de 30 minutes à 5 heures, de préférence de 30 minutes à 3 heures, et préférentiellement de 40 minutes à 80 minutes.

Selon un mode de réalisation préféré du procédé de l'invention, le temps de séjour de l'amine et de l'acide dans le réacteur (R2) est compris de 30 minutes à 5 heures, de préférence de 30 minutes à 3 heures, et préférentiellement de 40 minutes à 120 minutes.

De façon particulièrement avantageuse, l'acide utilisé dans le cadre du procédé de préparation de l'invention est l'acide monochloroacétique.

De façon particulièrement avantageuse, l'amine utilisée dans le cadre du procédé de préparation de l'invention est la lauryldiméthylamine.

Ainsi, un procédé particulièrement avantageux consiste à faire réagir de la lauryldiméthylamine avec de l'acide monochloroacétique en présence de potasse et d'eau dans un dispositif constitué de deux réacteurs tubulaires.

### DESCRIPTION DES FIGURES

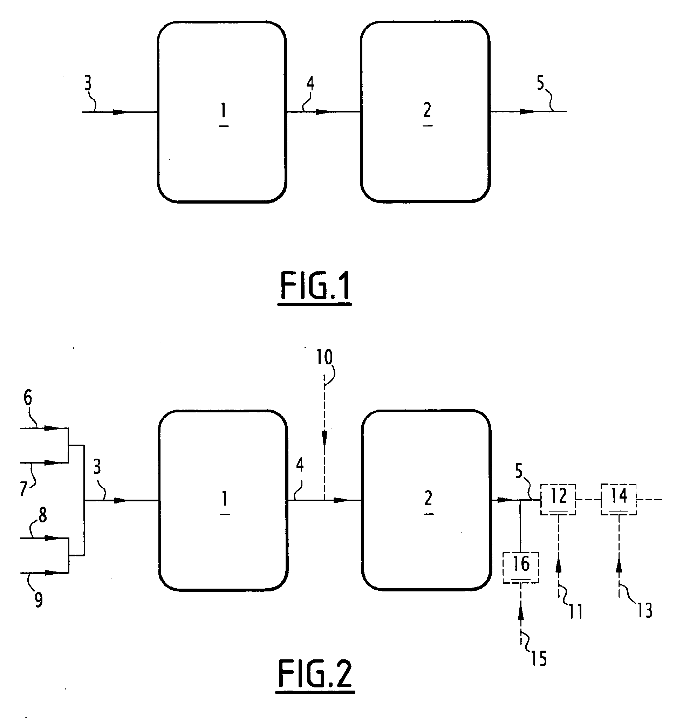

La Figure 1 représente un dispositif constitué de deux réacteurs (1) et (2) disposés en série, c'est-à-dire reliés entre eux. La sortie du réacteur (1) est reliée par l'intermédiaire de (4) à l'entrée du réacteur (2). A la sortie (5) du réacteur (2), on peut récupérer la solution aqueuse de bétaïne selon le procédé de la présente invention. La sortie (5) peut, le cas échéant, soit être reliée directement à des moyens destinés à charger des camions pour la livraison desdites solutions, soit être reliée à des moyens destinés à préparer des formulations à base de solutions aqueuses de bétaïne (ex. des mélangeurs).
   Les réactifs sont introduits en (3) dans le réacteur (1) et le mélange réactionnel (M) formé est récupéré en (4) à la sortie du réacteur (1) pour être réinjecté dans le réacteur (2).
La Figure 2 représente une variante du dispositif de la Figure 1.
   Ce dispositif est constitué de deux réacteurs (1) et (2) reliés entre eux par (4) (la sortie du réacteur (1) étant reliée à l'entrée du réacteur (2)). Les réactifs sont introduits à l'entrée (3) du réacteur (1). L'acide (6) et l'eau (7) sont introduits ensemble, d'une part, et l'amine (8) et la base (9) d'autre part.
   La sortie du réacteur (2) est reliée à un ou plusieurs mélangeurs (12, 14, 16) afin de préparer des formulations comprenant des solutions aqueuses de bétaïne (5) et d'autres composés (11, 13, 15).
   Ce dispositif comprend également une addition intermédiaire de base (10) entre la sortie du réacteur (1) et l'entrée du réacteur (2).

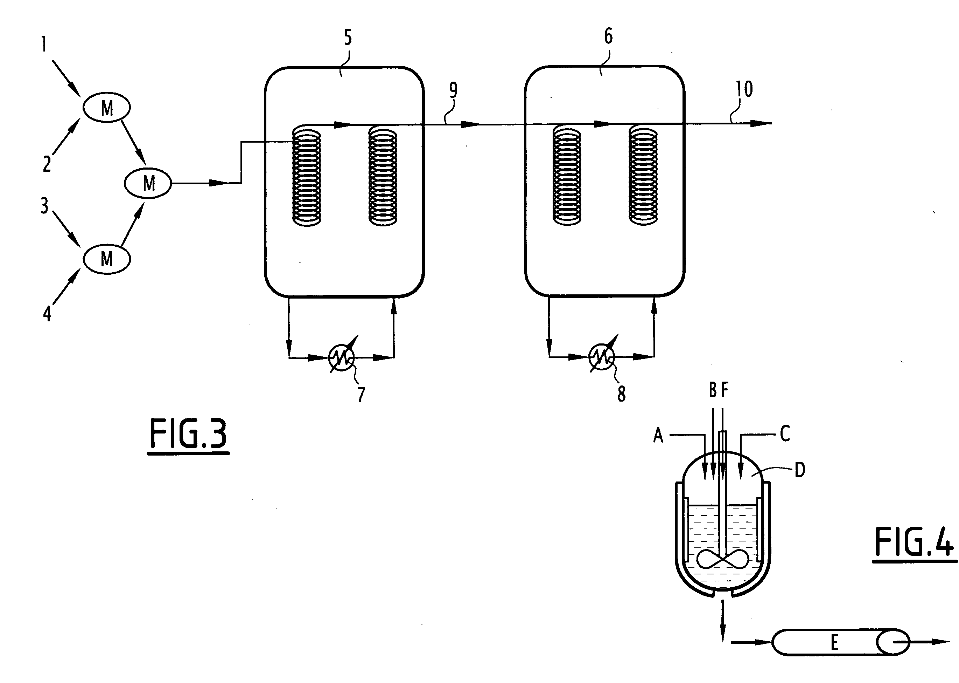

La Figure 3 représente une variante du dispositif de la Figure 1.
   L'eau (1) et l'acide (2), d'une part, et l'amine (3) et la base (4), d'autre part, sont introduits par l'intermédiaire de mélangeurs dans le premier réacteur tubulaire (5) comprenant des moyens de chauffage (7). La sortie du premier réacteur et l'entrée du deuxième réacteur tubulaire (6) sont reliées entre elles (9). Le deuxième réacteur (6) est muni de moyens de chauffage (8). A la sortie du réacteur (6), on récupère la solution aqueuse de bétaïne (10).
La Figure 4 représente une autre variante du dispositif de la Figure 1.
   Ce dispositif est constitué d'un premier réacteur agité (D) et d'un deuxième réacteur tubulaire (E). Les différents réactifs (A, B, C)(acide, amine et base) sont introduits directement en continu dans le réacteur (D) muni de moyens d'agitation (F).

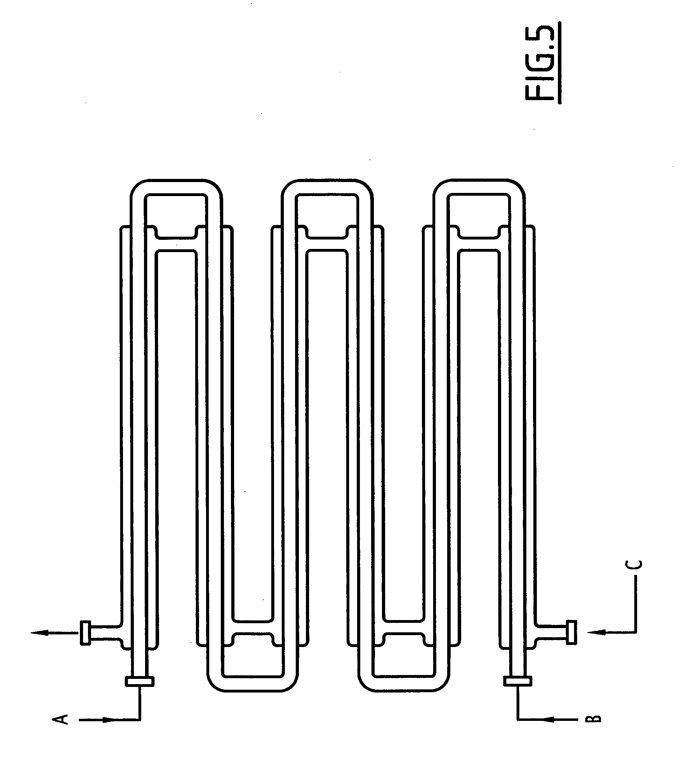

La Figure 5 représente un schéma détaillé d'un réacteur tubulaire.
   Les réactifs (A) sont introduits dans le réacteur tubulaire et les produits finals, notamment la solution aqueuse de bétaïne, sont récupérés à la sortie (B).
   Ce réacteur est muni de moyens de chauffage (C) par l'intermédiaire d'un fluide caloporteur.

### EXEMPLES

### Description générale du procédé

Le procédé de la présente invention a consisté à démarrer les pompes et les régler au débit souhaité, démarrer le bain chauffant de façon à maintenir les réacteurs en température. Une fois que le réacteur était à température, on a attendu 2 fois le temps de séjour total, environ 4 h, afin de stabiliser l'installation et des échantillonnages (en général 4 espacé de 30 min) ont été effectués, de façon à vérifier la reproductibilité des mesures.

Dans le cas de la mise en oeuvre de deux réacteurs tubulaires (R1) et (R2), on a mélangé l'amine LDMA avec la potasse contenant 1% de bétaïne. Le mélange résultant a ensuite été mélangé avec l'acide monochloroacétique avant d'entrer dans le réacteur (R1) puis le réacteur R2.

Dans le cas de la combinaison réacteur agité (R1) et réacteur tubulaire (R2), chaque constituant a été injecté séparément dans le réacteur agité. Chaque injection est assimilable à un tube plongeant qui libère le produit près de l'agitateur de façon à être bien mélangé. Le flux sortant s'écoule par surverse et est récolté dans un petit bac tampon qui alimente une pompe qui refoule le liquide dans le réacteur tubulaire (R2).

Les différents réactifs (amine, acide, base et eau) ont été introduits dans les réacteurs au moyen de pompes de chromatographie Gilson.

Les mélangeurs du dispositif de invention sont des micromélangeurs du fabricant IMM.

Les réacteurs tubulaires qui ont été utilisés sont des tubes de chromatographies de 1/8 ou 1/16 de pouce de diamètres internes et leur longueur a été ajustée en fonction du temps de séjour. On a enroulé ce tube de façon à obtenir des spires pratiquement jointives et un enroulement compact. Cet enroulement a été enfermé dans une enceinte dans laquelle circule un fluide caloporteur permettant de chauffer/refroidir les tubes. La circulation du fluide caloporteur et le contrôle de sa température ont été assurés grâce à un bain thermostaté à circulation. Chaque enceinte contenait deux enroulements reliés entre eux par une vanne trois voies à l'extérieure de l'enceinte, permettant de faire des échantillons ou une injection.

### EXEMPLE 1

Cet exemple a été effectué dans un dispositif constitué de deux réacteurs tubulaires (selon le schéma de la Figure 3).

La lauryldiméthylamine (LDMA) a été introduite avec un débit (Q1) de 0,41 mL/min et la potasse (base)(à 30%) a été introduite, en association avec 1% de bétaïne, avec un débit (Q2) de 0,26 mL/min. En parallèle, l'acide monochloroacétique (MCA)(à 28%) a été introduit avec un débit (Q3) de 0,505 mL/min.

Tous ces réactifs ont été introduits en continu dans le premier réacteur tubulaire. On a également introduit de la potasse (30%) avec un débit (Q4) de 0,08 mL/min entre la sortie du premier réacteur et l'entrée du second réacteur.

Le premier réacteur tubulaire a été maintenu à une température T1 égale à 95°C et le temps de séjour (t1) des réactifs était de 41 minutes. Le second réacteur tubulaire a été maintenu à une température T2 égale à 105°C et le temps de séjour (t2) des réactifs était de 82 minutes.

On a alors obtenu à la sortie du second réacteur une solution aqueuse de bétaïne comprenant 0,4% en poids de MCA résiduel, 1,5% en poids d'amine résiduelle et 1,6% en poids d'acide glycolique.

Une telle solution aqueuse répond aux spécifications pour une utilisation dans une composition herbicide.

### EXEMPLE 2

Cet exemple a été effectué dans le même dispositif que celui de l'exemple 1.

Les conditions étaient identiques en terme de débits mais les conditions de température étaient différentes :

| Q1 (mL/min) | Q2 (mL/min) | Q3 (mL/min) | Q4 (mL/min) | T1 (°C) | t1 (min) | T2 (°C) | t2 (min) |
|---|---|---|---|---|---|---|---|
| 0,41 | 0,26 | 0,505 | 0,08 | 100 | 41 | 100 | 82 |

On a alors obtenu à la sortie du second réacteur une solution aqueuse de bétaïne présentant les caractéristiques suivantes :

| MCA résiduel (% en poids) | amine résiduelle (% en poids) | acide glycolique (% en poids) |
|---|---|---|
| 0,26 | 2 | 1,6 |

### EXEMPLE 3

Cet exemple a été effectué dans le même dispositif que celui de l'exemple 1.

Les conditions étaient identiques en terme de débits mais les conditions de température étaient différentes :

| Q1 (mL/min) | Q2 (mL/min) | Q3 (mL/min) | Q4 (mL/min) | T1 (°C) | t1 (min) | T2 (°C) | t2 (min) |
|---|---|---|---|---|---|---|---|
| 0,41 | 0,26 | 0,505 | 0,08 | 95 | 41 | 100 | 82 |

On a alors obtenu à la sortie du second réacteur une solution aqueuse de bétaïne présentant les caractéristiques suivantes :

| MCA résiduel (% en poids) | amine résiduelle (% en poids) | acide glycolique (% en poids) |
|---|---|---|
| 0,56 | 1,8 | 1,4 |

### EXEMPLE 4

Cet exemple a été effectué dans le même dispositif que celui de l'exemple 1.

Les conditions étaient identiques en terme de débits mais les conditions de température étaient différentes :

| Q1 (mL/min) | Q2 (mL/min) | Q3 (mL/min) | Q4 (mL/min) | T1 (°C) | t1 (min) | T2 (°C) | t2 (min) |
|---|---|---|---|---|---|---|---|
| 0,41 | 0,26 | 0,505 | 0,08 | 95 | 41 | 95 | 82 |

On a alors obtenu à la sortie du second réacteur une solution aqueuse de bétaïne présentant les caractéristiques suivantes :

| MCA résiduel (% en poids) | amine résiduelle (% en poids) | acide glycolique (% en poids) |
|---|---|---|
| 0,7 | 2 | 1,3 |

### EXEMPLE 5

Cet exemple a été effectué dans le même dispositif que celui de l'exemple 1.

Les conditions étaient identiques en terme de débits mais les conditions de température et de temps de séjour étaient différentes :

| Q1 (mL/min) | Q2 (mL/min) | Q3 (mL/min) | Q4 (mL/min) | T1 (°C) | t1 (min) | T2 (°C) | t2 (min) |
|---|---|---|---|---|---|---|---|
| 0,41 | 0,26 | 0,505 | 0,08 | 130 | 61 | 130 | 61 |

On a alors obtenu à la sortie du second réacteur une solution aqueuse de bétaïne présentant les caractéristiques suivantes :

| MCA résiduel (% en poids) | amine résiduelle (% en poids) | acide glycolique (% en poids) |
|---|---|---|
| 0 | 8,1 | 4,3 |

On constate donc que lorsque les températures des réacteurs sont plus élevées (supérieures à 110 °C), on obtient des solutions de bétaïne avec une teneur importante en amine résiduelle.

De telles solutions ne sont pas adaptées pour une utilisation dans des compositions herbicides.

### EXEMPLE 6

Cet exemple a été effectué dans le même dispositif que celui de l'exemple 1, selon les conditions suivantes :

| Q1 (mL/min) | Q2 (mL/min) | Q3 (mL/min) | Q4 (mL/min) | T1 (°C) | t1 (min) | T2 (°C) | t2 (min) |
|---|---|---|---|---|---|---|---|
| 0,3 | 0,18 | 0,36 | 0,06 | 95 | 83 | 95 | 83 |

On a alors obtenu à la sortie du second réacteur une solution aqueuse de bétaïne présentant les caractéristiques suivantes :

| MCA résiduel (% en poids) | amine résiduelle (% en poids) | acide glycolique (% en poids) |
|---|---|---|
| 0,2 | 2,12 | 1,4 |

### EXEMPLE 7

Cet exemple a été effectué dans le même dispositif que celui de l'exemple 1, selon les conditions suivantes :

| Q1 (mL/min) | Q2 (mL/min) | Q3 (mL/min) | Q4 (mL/min) | T1 (°C) | t1 (min) | T2 (°C) | t2 (min) |
|---|---|---|---|---|---|---|---|
| 0,3 | 0,24 | 0,36 | 0 | 95 | 83 | 95 | 83 |

Ainsi, cet exemple a été effectué sans ajout intermédiaire de potasse (entre la sortie du premier réacteur et l'entrée du second réacteur).

On a alors obtenu à la sortie du second réacteur une solution aqueuse de bétaïne présentant les caractéristiques suivantes :

| MCA résiduel (% en poids) | amine résiduelle (% en poids) | acide glycolique (% en poids) |
|---|---|---|
| 0 | 8,3 | 3,4 |

On constate donc que l'ajout intermédiaire de potasse permet de réduire la teneur en amine résiduelle dans la solution finale de bétaïne.

Les solutions obtenues selon le procédé comprenant un tel ajout présentent des caractéristiques plus intéressantes pour une utilisation dans des compositions herbicides.

### EXEMPLE 8

Cet exemple a été effectué dans le dispositif de la Figure 4, à savoir un dispositif comprenant un réacteur agité et un réacteur tubulaire.

La lauryldiméthylamine (LDMA) a été introduite avec un débit (Q1) de 1,3 mL/min et la potasse (base)(à 45%) a été introduite avec un débit (Q2) de 0,55 mL/min. En parallèle, l'acide monochloroacétique (MCA)(à 19,9%) a été introduit avec un débit (Q3) de 2,24 mL/min.

Le premier réacteur (réacteur agité) a été maintenu à une température T1 égale à 95°C et le temps de séjour (t1) des réactifs était de 140 minutes. Le second réacteur tubulaire a été maintenu à une température T2 égale à 95°C et le temps de séjour (t2) des réactifs était de 60 minutes.

On a alors obtenu à la sortie du second réacteur une solution aqueuse de bétaïne comprenant 1,4% en poids de MCA résiduel, 0,9% en poids d'amine résiduelle et 2,31 % en poids d'acide glycolique.

### EXEMPLE 9

Cet exemple a été effectué dans le même dispositif que celui de l'exemple 8, selon les conditions suivantes :

| Q1 (mL/min) | Q2 (mL/min) | Q3 (mL/min) | T1 (°C) | t1 (min) | T2 (°C) | t2 (min) |
|---|---|---|---|---|---|---|
| 1,3 | 0,6 | 2,24 | 95 | 140 | 95 | 90 |

On a alors obtenu à la sortie du second réacteur une solution aqueuse de bétaïne présentant les caractéristiques suivantes :

| MCA résiduel (% en poids) | amine résiduelle (% en poids) | acide glycolique (% en poids) |
|---|---|---|
| 1,55 | 0,85 | 1,3 |

### EXEMPLE 10

Cet exemple a été effectué dans le même dispositif que celui de l'exemple 8, selon les conditions suivantes :

| Q1 (mL/min) | Q2 (mL/min) | Q3 (mL/min) | T1 (°C) | t1 (min) | T2 (°C) | t2 (min) |
|---|---|---|---|---|---|---|
| 1,3 | 0,63 | 2,24 | 95 | 140 | 95 | 75 |

On a alors obtenu à la sortie du second réacteur une solution aqueuse de bétaïne présentant les caractéristiques suivantes :

| MCA résiduel (% en poids) | amine résiduelle (% en poids) | acide glycolique (% en poids) |
|---|---|---|
| 1,2 | 1,65 | 1,2 |

### EXEMPLE 11

Cet exemple a été effectué dans le même dispositif que celui de l'exemple 8, selon les conditions suivantes :

| Q1 (mL/min) | Q2 (mL/min) | Q3 (mL/min) | T1 (°C) | t1 (min) | T2 (°C) | t2 (min) |
|---|---|---|---|---|---|---|
| 0,65 | 0,32 | 1,12 | 95 | 275 | 95 | 40 |

On a alors obtenu à la sortie du second réacteur une solution aqueuse de bétaïne présentant les caractéristiques suivantes :

| MCA résiduel (% en poids) | amine résiduelle (% en poids) | acide glycolique (% en poids) |
|---|---|---|
| 0,7 | 1,32 | 1,37 |

Une telle solution aqueuse répond aux spécifications pour une utilisation dans une composition herbicide.

## Revendications

1. Procédé de préparation en continu d'une solution aqueuse de bétaïne de formule (I) suivante : 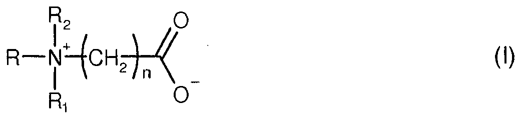 dans laquelle :
- n est égal à 1, 2 ou 3 ;
- R₁ représente un groupe alkyle, linéaire ou ramifié, comprenant de 1 à 3 atomes de carbone, de préférence un groupe méthyle ;
- R₂ représente un groupe alkyle, linéaire ou ramifié, comprenant de 1 à 3 atomes de carbone, de préférence un groupe méthyle ;
- R représente :
. soit une chaîne hydrocarbonée, linéaire ou ramifiée, de préférence un groupe alkyle, comprenant de 3 à 30 atomes de carbone, et notamment de 3 à 20 atomes de carbone ;
. soit un groupe -A-NH-CO-R₃,
R₃ représentant une chaîne hydrocarbonée, linéaire ou ramifiée, de préférence un groupe alkyle, comprenant de 3 à 30 atomes de carbone, et notamment de 3 à 20 atomes de carbone ; et
A représentant un groupe hydrocarboné divalent, linéaire ou ramifié, comprenant de 1 à 6 atomes de carbone, éventuellement substitué par un groupe hydroxyle, et de préférence choisi parmi les groupes : -CH₂-CH₂-CH₂- et -CH₂-CHOH-CH₂- ;
comprenant la réaction d'une amine de formule NRR¹R², R, R¹ et R² étant tels que définis ci-dessus, avec un acide ω-halocarboxylique de formule X-(CH₂)ₙ-COOH, X représentant un atome d'halogène et n étant tel que défini ci-dessus, en présence d'eau et d'une base, notamment d'un hydroxyde de métal alcalin, et plus particulièrement KOH ou NaOH,
ledit procédé étant **caractérisé en ce qu'**il est effectué dans un dispositif constitué d'au moins deux réacteurs successifs (R1) et (R2), le réacteur (R2) étant un réacteur tubulaire.

2. Procédé selon la revendication 1, comprenant :
a) une étape d'introduction, à l'entrée du réacteur (R1), de l'eau, de la base, de l'amine et de l'acide ω-halocarboxylique ;
b) une étape de réaction entre l'amine et l'acide ω-halocarboxylique dans le réacteur (R1), pour obtenir, à la sortie du réacteur (R1), un mélange réactionnel aqueux (M) comprenant de la bétaïne, de l'amine et de l'acide ω-halocarboxylique ;
c) une étape d'introduction du mélange réactionnel aqueux (M) à l'entrée du réacteur (R2), pour poursuivre la réaction entre l'amine et l'acide ω-halocarboxylique ; et
d) une étape de récupération d'une solution aqueuse de bétaïne (M') à la sortie du réacteur (R2) comprenant de la bétaïne à raison d'au moins 20%, et de préférence d'au moins 30%, en poids, et moins de 2,5% en poids, de préférence moins de 1,65% en poids, d'amine, moins de 1,5% en poids, notamment moins de 1% en poids, et de préférence moins de 0,7% en poids, d'acide.

3. Procédé selon la revendication 2, **caractérisé en ce que**, lors de l'étape a), on introduit, d'une part, l'amine avec la base, et, d'autre part, l'acide avec l'eau.

4. Procédé selon la revendication 2 ou 3, **caractérisé en ce qu'**il comprend une étape supplémentaire b'), consistant à ajouter de la base dans le mélange réactionnel (M) à la sortie du réacteur (R1) et avant l'introduction dudit mélange dans le réacteur (R2).

5. Procédé selon l'une quelconque des revendications 2 à 4, **caractérisé en ce qu'**une portion de la solution aqueuse de bétaïne (M') est récupérée à la sortie du réacteur (R2) pour être injectée à l'entrée du réacteur (R1) avec l'amine.

6. Procédé selon l'une quelconque des revendications 1 à 5, dans lequel la sortie du réacteur (R2) est reliée à un ou plusieurs mélangeurs comprenant de l'eau, de la glycérine, des agents anti-mousse ou des mélanges de ceux-ci, afin de préparer des formulations en ligne à base de solution aqueuse de bétaïne.

7. Procédé de préparation selon l'une quelconque des revendications 1 à 6, dans lequel la température à l'intérieur du réacteur (R1) est comprise de 80 °C à 110°C, de préférence de 90°C à 100°C, et préférentiellement égale à 95°C.

8. Procédé de préparation selon l'une quelconque des revendications 1 à 7, dans lequel la température à l'intérieur du réacteur (R2) est comprise de 95 °C à 110 °C, de préférence de 100 °C à 105 °C, et préférentiellement égale à 105 °C.

9. Procédé de préparation selon l'une quelconque des revendications 1 à 8, dans lequel la pression à l'intérieur du réacteur (R2) est comprise de 1 à 10 bars, de préférence de 1,5 à 5 bars, et préférentiellement comprise entre 2 et 3 bars.

10. Procédé de préparation selon l'une quelconque des revendications 1 à 9, **caractérisé en ce que** le réacteur (R1) est un réacteur tubulaire.

11. Procédé de préparation selon l'une quelconque des revendications 1 à 10, **caractérisé en ce que** le temps de séjour de l'amine et de l'acide dans le réacteur (R1) est compris de 30 minutes à 5 heures, de préférence de 30 minutes à 3 heures, et préférentiellement de 40 minutes à 80 minutes.

12. Procédé de préparation selon l'une quelconque des revendications 1 à 11, **caractérisé en ce que** le temps de séjour de l'amine et de l'acide dans le réacteur (R2) est compris de 30 minutes à 5 heures, de préférence de 30 minutes à 3 heures, et préférentiellement de 40 minutes à 120 minutes.

13. Procédé de préparation selon l'une quelconque des revendications 1 à 12, dans lequel l'acide est l'acide monochloroacétique.

14. Procédé de préparation selon l'une quelconque des revendications 1 à 13, dans lequel l'amine est la lauryldiméthylamine.

## Patentansprüche

1. Verfahren zur kontinuierlichen Herstellung einer wässrigen Lösung von Betain nach der folgenden Formel (I): 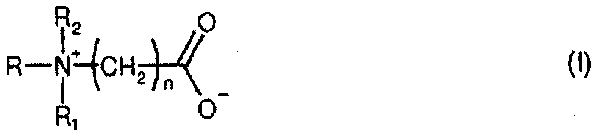 wobei:
- n 1, 2 oder 3 ist;
- R₁ eine lineare oder verzweigte Alkylgruppe mit 1 bis 3 Kohlenstoffatomen, bevorzugt eine Methylgruppe, darstellt;
- R₂ eine lineare oder verzweigte Alkylgruppe mit 1 bis 3 Kohlenstoffatomen, bevorzugt eine Methylgruppe, darstellt;
- R
• eine lineare oder verzweigte Kohlenwasserstoffkette, bevorzugt eine Alkylgruppe mit 3 bis 30 Kohlenstoffatomen, besonders bevorzugt 3 bis 20 Kohlenstoffatomen, darstellt;
• eine -A-NH-CO-R₃-Gruppe darstellt, wobei R₃ eine lineare oder verzweigte Kohlenwasserstoffkette, bevorzugt eine Alkylgruppe mit 3 bis 30 Kohlenstoffatomen, besonders bevorzugt mit 3 bis 20 Kohlenstoffatomen, darstellt; und
A eine zweiwertige, lineare oder verzweigte Kohlenwasserstoffgruppe mit 1 bis 6 Kohlenstoffatomen darstellt, optional substituiert mit einer Hydroxyl-Gruppe, und bevorzugt ausgewählt ist aus der Gruppe bestehend aus -CH₂-CH₂-CH₂- und -CH₂-CHOH-CH₂-;
umfassend das Umsetzen eines Amins der Formel NRR¹R², wobei R, R¹ und R² wie oben definiert sind, mit einer ω-Halogencarbonsäure der Formel X-(CH₂)ₙ-COOH, wobei X ein Halogenatom darstellt und n wie oben definiert ist, in Gegenwart von Wasser und einer Base, bevorzugt einem Alkalimetallhydroxid, besonders bevorzugt NaOH oder KOH,
wobei das Verfahren **dadurch gekennzeichnet ist, dass** es in einer Vorrichtung bestehend aus mindestens zwei aufeinanderfolgenden Reaktoren (R1) und (R2) durchgeführt wird, wobei der Reaktor (R2) ein Rohrreaktor ist.

2. Verfahren nach Anspruch 1, umfassend:
a) einen Einführungsschritt am Eingang des Reaktors (R1) von Wasser, der Base, des Amins und der ω-Halogencarbonsäure;
b) einen Reaktionsschritt des Amins mit der ω-Halogencarbonsäure im Reaktor (R1), um am Ausgang des Reaktors (R1) ein wässriges Reaktionsgemisch (M) umfassend das Betain, das Amin und die ω-Halogencarbonsäure zu erhalten;
c) einen Einführungsschritt des wässrigen Reaktionsgemischs (M) am Eingang des Reaktors (R2), um die Reaktion des Amins mit der ω-Halogencarbonsäure fortzusetzen; und
d) einen Rückgewinnungsschritt einer wässrigen Lösung von Betain (M') am Ausgang des Reaktors (R2), umfassend eine Menge von mindestens 20 Gew.%, bevorzugt von mindestens 30 Gew.% Betain, weniger als 2,5 Gew.%, bevorzugt weniger als 1,65 Gew.% Amin, und weniger als 1,5 Gew.%, bevorzugt weniger als 1 Gew.% und besonders bevorzugt weniger als 0,7 Gew.% Säure.

3. Verfahren nach Anspruch 2, wobei während des Schritts a) das Amin mit der Base als ein Teil und die Säure mit Wasser als der andere Teil eingeführt werden.

4. Verfahren nach Anspruch 2 oder 3, wobei das Verfahren einen zusätzlichen Schritt b') umfasst, der in der Zugabe der Base zum Reaktionsgemisch (M) am Ausgang des Reaktors (R1) und vor dem Einführen des Gemischs in den Reaktor (R2) besteht.

5. Verfahren nach einem der Ansprüche 2 bis 4, wobei ein Teil der wässrigen Lösung des Betains (M') am Ausgang des Reaktors (R2) wiedererhalten wird, um am Eingang des Reaktors (R1) mit dem Amin injiziert zu werden.

6. Verfahren nach einem der Ansprüche 1 bis 5, wobei der Ausgang des Reaktors (R2) mit einem oder mehreren Mischern verbunden ist, die Wasser, Glycerin, Antischaummittel oder Gemische davon enthalten, um Formulierungen auf der Basis einer wässrigen Lösung von Betain herzustellen.

7. Verfahren nach einem der Ansprüche 1 bis 6, wobei die Temperatur im Inneren des Reaktors (R1) 80 °C bis 110 °C, bevorzugt 90 °C bis 100 °C und besonders bevorzugt 95 °C beträgt.

8. Verfahren nach einem der Ansprüche 1 bis 7, wobei die Temperatur im Inneren des Reaktors (R2) 95 °C bis 110 °C, bevorzugt 100 °C bis 105 °C und besonders bevorzugt 105 °C beträgt.

9. Verfahren nach einem der Ansprüche 1 bis 8, wobei der Druck im Inneren des Reaktors (R2) von 1 bis 10 bar, bevorzugt 1,5 bis 5 bar und besonders bevorzugt zwischen 2 und 3 bar beträgt.

10. Verfahren nach einem der Ansprüche 1 bis 9, wobei der Reaktor (R1) ein Rohrreaktor ist.

11. Verfahren nach einem der Ansprüche 1 bis 10, wobei die Verweildauer des Amins und der Säure im Reaktor (R1) zwischen 30 Minuten bis 5 Stunden, bevorzugt von 30 Minuten bis 3 Stunden und besonders bevorzugt von 40 Minuten bis 80 Minuten beträgt.

12. Verfahren nach einem der Ansprüche 1 bis 11, wobei die Verweildauer des Amins und der Säure im Reaktor (R2) zwischen 30 Minuten bis 5 Stunden, bevorzugt von 30 Minuten bis 3 Stunden und besonders bevorzugt von 40 Minuten bis 120 Minuten beträgt.

13. Verfahren nach einem der Ansprüche 1 bis 12, wobei die Säure mono-Chloressigsäure ist.

14. Verfahren nach einem der Ansprüche 1 bis 13, wobei das Amin Lauryldimethylamin ist.

## Claims

1. A continuous method for preparing an aqueous solution of betaine having the following formula (I): 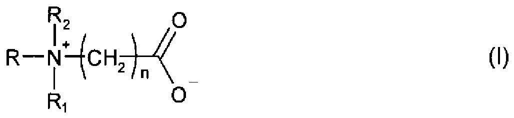 wherein:
- n is equal to 1, 2 or 3;
- R₁ represents a linear or branched alkyl group, comprising from 1 to 3 carbon atoms, preferably a methyl group;
- R₂ represents a linear or branched alkyl group, comprising from 1 to 3 carbon atoms, preferably a methyl group;
- R represents:
. either a linear or branched hydrocarbonaceous chain, preferably an alkyl group, comprising from 3 to 30 carbon atoms, and in particular from 3 to 20 carbon atoms;
. or a -A-NH-CO-R₃ group,
R₃ representing a linear or branched hydrocarbonaceous chain, preferably an alkyl group, comprising from 3 to 30 carbon atoms, and in particular from 3 to 20 carbon atoms; and
A representing a linear or branched divalent hydrocarbonaceous group comprising from 1 to 6 carbon atoms, possibly substituted by a hydroxyl group, and preferably chosen from among the groups: -CH₂-CH₂-CH₂-and -CH₂-CHOH-CH₂-;
comprising the reaction of an amine of formula NRR¹R², R, R¹ and R² being as defined above, with a ω-halocarboxylic acid having formula X-(CH₂)ₙ-COOH, X representing a halogen atom and n being as defined above, in the presence of water and a base, in particular an alkali metal hydroxide, and more particularly KOH or NaOH,
said method being **characterized in that** said method is carried out in a device made up of at least two consecutive reactors (R1) and (R2), the reactor (R2) being a tubular reactor.

2. The method according to claim 1, comprising:
a) a step for introducing water, base, amine, and the ω-halocarboxylic acid at the inlet of the reactor (R₁),
b) a reaction step between the amine and the ω-halocarboxylic acid in the reactor (R1) to obtain, at the outlet of the reactor (R1), an aqueous reactive mixture (M) comprising betaine, amine and ω-halocarboxylic acid;
c) a step for introducing the aqueous reactive mixture (M) at the inlet of the reactor (R2), to continue the reaction between the amine and the ω-halocarboxylic acid; and
d) a step for recovering an aqueous solution of betaine (M') at the outlet of the reactor (R2) comprising at least 20 wt% of betaine, and preferably at least 30% by weight; and less than 2.5 wt% by weight, preferably less than 1.65 wt% by weight, of amine; and less than 1.5 wt% by weight, in particular less than 1 wt% by weight, and preferably less than 0.7 wt% by weight, of acid.

3. The method according to claim 2, **characterized in that**, during step a), the amine is introduced with the base on the one hand, and the acid is introduced with the water on the other hand.

4. The method according to claim 2 or 3, **characterized in that** it comprises an additional step b'), consisting of adding base into the reactive mixture (M) at the outlet of the reactor (R1) and before introducing said mixture into the reactor (R2).

5. The method according to any one of claims 2 to 4, **characterized in that** a portion of the aqueous solution of betaine (M') is recovered at the outlet of the reactor (R2) to be injected at the inlet of the reactor (R1) with the amine.

6. The method according to any one of claims 1 to 5, wherein the outlet of the reactor (R2) is connected to one or more mixers comprising water, glycerin, antifoaming agents or mixtures thereof, in order to prepare betaine aqueous solution-based in-line formulations.

7. The method according to any one of claims 1 to 6, wherein the temperature inside the reactor (R1) is comprised from 80°C to 110°C, preferably from 90°C to 100°C, and preferably equal to 95°C.

8. The method according to any one of claims 1 to 7, wherein the temperature inside the reactor (R2) is comprised from 95°C to 110°C, preferably from 100°C to 105°C, and preferably equal to 105°C.

9. The method according to any one of claims 1 to 8, wherein the pressure inside the reactor (R2) is comprised from 1 to 10 bars, preferably from 1.5 bars to 5 bars, and preferably from 2 to 3 bars.

10. The method according to any one of claims 1 to 9, wherein the reactor (R1) is a tubular reactor.

11. The method according to any one of claims 1 to 10, wherein the residence time of the amine and the acid in the reactor (R1) is comprised from 30 minutes to 5 hours, preferably from 30 minutes to 3 hours, and preferably from 40 minutes to 80 minutes.

12. The method according to any one of claims 1 to 11, **characterized in that** the residence time of the amine and the acid in the reactor (R2) is comprised from 30 minutes to 5 hours, preferably from 30 minutes to 3 hours, and preferably from 40 minutes to 120 minutes.

13. The method according to any one of claims 1 to 12, wherein the acid is monochloroacetic acid.

14. The method according to any one of claims 1 to 13, wherein the amine is lauryldimethylamine.
